## Europäisches Patentamt

## European Patent Office

## Office européen des brevets

(11) Numéro de publication: **0 178 957**
**B1**

(12) **FASCICULE DE BREVET EUROPEEN**

(45) Date de publication du fascicule du brevet:
31.01.90

(51) Int. Cl.⁴: **C 12 N 9/36, A 23 L 1/32**

(21) Numéro de dépôt: 85401688.8

(22) Date de dépôt: 27.08.85

(54) Procédé pour l'obtention de lysozyme par microfiltration à partir d'une matière à base de blanc d'oeuf.

(30) Priorité: 28.08.84 FR 8413305

(43) Date de publication de la demande:
23.04.86 Bulletin 86/17

(45) Mention de la délivrance du brevet:
31.01.90 Bulletin 90/5

(84) Etats contractants désignés:
AT BE CH DE FR GB IT LI LU NL SE

(56) Documents cités:
FR-A- 2 157 826

CHEMICAL ABSTRACTS, vol. 96, no. 9, 1 mars 1982,
page 251, no. 64850s, Columbus, Ohio, US; L.V.
KRYLOVA et al.: "Study of the effect of external factors
on the ultrafiltration of water-salt solutions of
lysozyme" & DEPOSITED DOC. 1980, VINITI 3601-80, 14
pp
CHEMICAL ABSTRACTS, vol. 94, no. 17, 27 avril 1981,
page 388, no. 135510c, Columbus, Ohio, US; K.C.
INGHAM et al.: "Separation of macromolecules by
ultrafiltration: influence of protein adsorption,
protein-protein interactions, and concentration
polarization" & POLYM. SCI. TECHNOL. 1980,

(73) Titulaire: INSTITUT NATIONAL DE LA RECHERCHE
AGRONOMIQUE, 149, Rue de Grenelle, F-75341 Paris
Cedex 07 (FR)

(72) Inventeur: Lepienne, Alain, 15 Faubourg La Grappe,
F-28000 Chantres (FR)
Inventeur: Maubois, Jean-Louis, La Barre, Guibourg,
F-35740 Pace (FR)
Inventeur: Thireau, Michel, Bellerive Taupon,
56800 Ploermel (FR)
Inventeur: Piot, Michel, 10 rue du Bourbonnais,
35100 Rennes (FR)

(74) Mandataire: Phélip, Bruno et al, c/o Cabinet Harlé &
Phélip 21, rue de La Rochefoucauld, F-75009 Paris (FR)

(56) Documents cités: (suite)
13(ULTRAFILTR. MEMBR. APPL.) 141-58
CHEMICAL ABSTRACTS, vol. 97, no. 19, 8 novembre
1982, page 348, no. 158929x, Columbus, Ohio, US; G.B.
TANNY et al.: "Biotechnical applications of a pleated
crossflow microfiltration module" & DESALINATION
1982, 41(3), 299-312
Evaporation, Membrane filtration and spray drying in
milk powder and cheese production (1985) R. Hansen
ed. North Europ. Dairy Journal, Denmark pp. 188
Ultrafiltration Handbook, M. Cheryan 1986, p. 56-59
Tecnomic Publ. Lancaster, Basel

## Description

La présente invention concerne un nouveau procédé de préparation de lysozyme ou muramidase (E.C.3.2.1.17) à partir d'une matière première à base de blanc d'œuf, utilisant la technique de microfiltration.

Le lysozyme est présent dans la plupart des liquides physiologiques (salive, larmes, lait de femme). La principale source industrielle de lysozyme est le blanc d'œuf de poule qui en contient 3 à 6 g/kg. Les propriétés enzymatiques de cette protéine lui confèrent une action bactéricide essentiellement sur les bactéries Gram+. Ses propriétés antibactériennes sont utilisées pour des applications préventives telles que la supplémentation des laits maternisés et des applications curatives sous forme de médicaments contre la toux, certaines affections buccales et autres. Depuis une dizaine d'années, le champ d'action du lysozyme s'est considérablement élargi du fait de son utilisation en industrie fromagère. Le lysozyme est en effet employé pour prévenir le développement de la flore butyrique des laits destinés à la fabrication d'Emmental.

Au sens de la présente invention, l'expression «matière première à base de blanc d'œuf» couvre le blanc d'œuf lui-même et/ou l'entier d'œuf aussi bien que les mélanges de ces produits avec divers additifs, ainsi qu'il sera décrit ci-après plus en détails.

L'entier d'œuf est un mélange de blanc et de jaune d'œuf en proportion variable (40 à 80% du blanc). La composition typique du jaune d'œuf industriel est la suivante:
eau 44–50; protéines 15,7–16,6; lipides 31,8–35,5; glucides 0,2–1; minéraux 1,1.

Le blanc d'œuf industriel ou coule fraîche est obtenu par la casse des œufs qui permet de libérer le contenu de l'œuf et de séparer le blanc d'œuf du jaune d'œuf. La composition moyenne en poids du blanc d'œuf industriel est la suivante: eau 88,5%; protéines 10,5; lipides traces; glucides 0,5; minéraux 0,5.

Les protéines représentent la fraction majeure de la matière sèche: le rapport matières azotées totales sur extrait sec est généralement supérieur à 90%. Le pH du blanc d'œuf frais, immédiatement après la casse, se situe entre 9 et 9,5. Le blanc d'œuf industriel est essentiellement utilisé en industrie biscuitière pour ses propriétés foisonnantes.

Une douzaine de protéines ont été identifiées dans le blanc d'œuf. Leurs teneurs et caractéristiques respectives ont également été précisées. Le lysozyme est une des protéines du blanc d'œuf et se différencie des autres protéines par deux caractéristiques essentielles:
– son très faible poids moléculaire (14 600 dalton) qui en fait la plus petite des protéines du blanc d'œuf;
– sa forte charge électropositive au pH du blanc d'œuf (son pH isoélectrique est, en effet, 10,7).

C'est précisément sur l'une ou l'autre de ces propriétés que reposent les procédés actuels d'extraction du lysozyme du blanc d'œuf. Il existe trois techniques principales dont deux sont utilisées industriellement.

Les techniques chromatographiques échangeuses d'ions utilisent les caractéristiques électriques du lysozyme. Le principe de ce type de procédé consiste à fixer le lysozyme sur des résines échangeuses de cations, puis à désorber le lysozyme à l'aide d'une solution d'élution. La nature de la résine peut être variable.

Un tel procédé permet d'obtenir un rendement d'extraction du lysozyme voisin de 70%, mais sa mise en œuvre est complexe et demande une haute technicité du personnel. De plus, il conduit à la production d'une quantité d'effluents polluants au moins égale à la quantité de blanc d'œuf traitée.

La seconde technique industrielle consiste à précipiter le lysozyme par salage du blanc d'œuf au voisinage de son pH isoélectrique (pH 9,5). Le rendement d'extraction est de l'ordre de 60%; le procédé est simple, mais il présente les inconvénients majeurs d'immobiliser le blanc d'œuf 72 h, et de fournir un sous-produit, le blanc d'œuf salé, dont les propriétés fonctionnelles (aptitude au foisonnement, en particulier) sont altérées et l'utilisation limitée (aux industries de la viande, surtout).

Enfin, la dernière technique consiste à effectuer un tamisage moléculaire des protéines du blanc d'œuf sur des membranes d'ultrafiltration. Ces membranes sont utilisées classiquement dans les industries agro-alimentaires pour concentrer et purifier les produits liquides avant séchage, en éliminant une partie de l'eau et des substances dissoutes qu'ils contiennent. Des documents illustratifs de la technique d'ultrafiltration dans le domaine du traitement du blanc d'œuf sont le brevet FR-A 2 157 826 et les articles cités dans Chemical Abstracts, vol. 96, n°9, 1er mars 1982, page 251, n° 64850s, Columbus, Ohio, US; L. V. Krylova et al.: «Study of the effect of external factors on the ultrafiltration of water-salt solutions of lysoszyme» & Deposited Doc. 1980, Viniti 3601-80, 14 pp. et Chemical Abstracts, vol. 4, n° 17, 27 avril 1981, page 388, n° 135510c, Columbus, Ohio, US; K. C. Ingham et al.: «Separation of macromolecules by ultrafiltration: influence of protein adsorption, protein-protein interactions, and concentration polarization» & Polym. Sci. Technol. 1980, 13 (Ultrafilter. Membr. Appl.) 141–58. Le faible rendement de la méthode (voisin de 25% du lysozyme du blanc d'œuf) empêche son extension industrielle.

La présente invention a pour objet un procédé qui remédie aux inconvénients de la technique antérieure. Elle concerne en particulier un procédé d'extraction du lysozyme à partir du blanc d'œuf et/ou d'entier d'œuf, procédé qui est avantageux à mettre en œuvre à l'échelle industrielle, et fournit notamment un haut rendement d'extraction tout en permettant d'obtenir à la fois un lysozyme de grande pureté et un entier d'œuf et/ou un blanc d'œuf appauvri en lysozyme, mais conservant toutes les propriétés du produit naturel.

L'invention a donc pour objet un procédé pour l'obtention de lysozyme à partir d'une matière première à base de blanc d'œuf, caractérisé en ce qu'on soumet une matière première fluide à base de blanc d'œuf, telle que définie dans la présente description, à au moins une microfiltration, par mise en contact de ladite matière sous pression et en flux tangentiel avec une membrane capable de séparer les particules ayant une taille comprise entre 0,02 micron et 10 microns environ, ce qui fournit d'une part un microfiltrat passant à travers la membrane et contenant au moins une fraction du lysozyme de la matière première et, d'autre part, un produit retenu par la membrane, qui conserve les propriétés fonctionnelles de la matière première à base de blanc d'œuf, mais est appauvri en lysozyme.

Ainsi, la présente invention consiste à appliquer la technique industrielle de microfiltration à l'extraction du lysozyme du blanc d'œuf et/ou d'entier d'œuf. La microfiltration est une technique connue de séparation ou d'élimination de particules: voir par exemple l'ouvrage de H.W. Ballew intitulé «Basics of filtration and separation», (1978). Selon cette technique, la matière de départ est mise en contact avec une membrane ayant certaines caractéristiques de porosité. Cette mise en contact se fait sous pression, en flux tangentiel, pour forcer une fraction du produit dénommé microfiltrat, à passer à travers la membrane. Ainsi que l'indique l'ouvrage de H.W. Ballew précité, la différence fondamentale qui existe entre l'ultrafiltration et la microfiltration est que l'ultrafiltration permet de séparer des molécules alors que la microfiltration a pour but la séparation de particules. Les deux techniques diffèrent donc essentiellement par la nature des membranes mises en œuvre. Dans le cas de la microfiltration, les membranes ont un diamètre de pore au moins cent fois supérieur à celui des membranes d'ultrafiltration. Contrairement à ces dernières, qui ne laissent passer théoriquement que l'eau et les substances dissoutes non protéiques, les membranes de microfiltration ne retiennent que les microorganismes, les substances colloïdales ou micellaires.

Les applications des membranes d'ultrafiltration sont les concentrations sélectives des matières protéiques des liquides agroalimentaires. Les principales applications de la microfiltration sont: l'épuration bactérienne à basse température et la clarification des liquides agroalimentaires.

La présente invention met à profit la technique générale de microfiltration pour extraire le lysozyme du blanc d'œuf ou/et d'entier d'œuf. Les résultats obtenus par le procédé sont particulièrement intéressants et surprenants. En premier lieu, l'extraction est parfaitement sélective, car le microfiltrat passant à travers la membrane contient au moins une partie du lysozyme de la matière première à base de blanc d'œuf ou d'entier d'œuf, à l'exclusion des autres protéines de celui-ci. Le produit retenu par la membrane est un blanc d'œuf ou un entier d'œuf ou un rétentat (produit d'œuf enrichi en protéines et éventuellement en lipides) exempt d'une partie de son lysozyme mais conservant toutes les propriétés du produit naturel (pH, propriétés foisonnantes, composition relative en protéines autres que le lysozyme).

Selon le procédé de l'invention, le rendement d'extraction du lysozyme du blanc d'œuf ou de l'œuf entier est de 50 à 80% ou davantage.

Contrairement à la technique connue de précipitation, l'invention permet de n'immobiliser le blanc d'œuf ou l'œuf entier traité que pendant une durée très courte, inférieure à 6 heures. Enfin, le procédé se prête parfaitement à une mise en œuvre industrielle en raison de l'extrême simplicité des conditions opératoires et du faible coût d'extraction qu'il procure.

Les conditions de mise en œuvre de la microfiltration n'ont pas de caractère critique. Les paramètres opératoires, tels que la température, la pression et la vitesse de circulation du produit dépendent uniquement du type de membranes utilisé. Celles-ci sont du type usuel en microfiltration et sont capables de séparer des particules ayant une taille comprise entre 0,02 micron et 10 microns environ. Ainsi qu'il est classique, les montages de membranes sont organisés sous forme de dispositifs dénommés modules. Il est en général avantageux de travailler à des températures supérieures à la température ordinaire afin de faciliter la mise en contact du produit fluide avec la membrane. Comme le montrent les exemples qui suivent, des températures de l'ordre de 40 à 45°C se sont avérées convenables. Le procédé implique le passage de la matière première fluide sur la membrane avec une pression positive. Il est, cependant, avantageux de limiter par des moyens hydrodynamiques connus la croissance en épaisseur et la densité de texture de la couche de polarisation, par exemple en faisant circuler le liquide à base de blanc d'œuf à une vitesse égale ou supérieure à 5 m/s et en limitant, autant que la conception du module le permet, la pression d'entrée. Comme l'illustrent les exemples suivants, des pressions inférieures à 5 bars et notamment de l'ordre de 2 à 3 bars se sont avérées appropriées.

Pour la mise en œuvre du procédé de l'invention, on peut utiliser directement le blanc d'œuf, mais on préfère le diluer au préalable avec de l'eau contenant un sel soluble, tel que le chlorure de sodium, ou encore un sucre soluble tel que le glucose ou le saccharose, de manière à obtenir à la fois une matière première fluide convenant à la microfiltration et un rendement plus élevé d'extraction du lysozyme. On peut également utiliser directement de l'entier d'œuf, mais on préfère le diluer au préalable avec de l'eau contenant un sel soluble, tel que le chlorure de sodium ou un sucre soluble, tel que le glucose ou le saccharose.

Selon la présente invention, une substance antioxydante, autorisée par la réglementation en vigueur, telle que l'acide ascorbique, peut être ajoutée préalablement à l'œuf entier pour éviter tout endommagement des lipides du produit au cours du traitement par microfiltration.

Ainsi qu'on l'a mentionné précédemment, tous les mélanges ainsi mis en œuvre entrent dans le cadre de la présente invention et constituent la matière première à base de blanc d'œuf, objet du procédé.

On notera également que, ainsi qu'il est usuel dans la technique de microfiltration, il est avantageux dans certains cas, bien que cela ne soit absolument pas nécessaire, de travailler par diafiltration du blanc d'œuf ou d'entier d'œuf, natures ou dilués, en ajoutant ainsi à la matière première une quantité d'eau égale à la quantité de microfiltrat sortant. Les exemples 1, 2, 4 et 5 ci-après sont illustratifs de cette technique.

Le blanc d'œuf ou l'entier d'œuf, ou les rétentats respectifs, délysozymés obtenus par le procédé ci-dessus décrit sont utilisables en l'état, sous forme liquide ou congelée, ou encore concentrés par évaporation, éventuellement supplémentés en additifs (sels, sucres, acide ascorbique) ou séchés. Le lysozyme contenu dans le microfiltrat peut être, selon un procédé connu, concentré et purifié par ultrafiltration sur membrane de porosité comprise entre 2000 et 10000 daltons, puis déshydraté par lyophilisation ou séchage par atomisation.

L'invention sera maintenant illustrée sans être aucunement limitée par les exemples qui suivent:

Exemple 1

7 kg de blanc d'œuf d'une teneur de 4,5 g/kg en lysozyme sont préalablement déchalazés et homogénéisés. Ils sont ensuite dilués dans 14 kg d'eau salée à 5 g/kg de chlorure de sodium. Le mélange est microfiltré à l'aide d'un dispositif de microfiltration comprenant un module de microfiltration SFEC équipé de membrans M 6-4 d'une surface égale à 0,113 m$^2$. Les conditions opératoires sont les suivantes: température de microfiltration de 40°C, pression d'entrée dans le module de microfiltration de 2 bars, vitesse de circulation dans le module de microfiltration de 1 à 8 m/s, préférablement 4 à 6 m/s.

On réalise, dès le début de la microfiltration, une diafiltration du blanc d'œuf dilué (ajout en continu d'une quantité d'eau égale à la quantité du microfiltrat sortant). La diafiltration dure 4 h puis on effectue la concentration du mélange pour obtenir les 7 kg de blanc d'œuf initiaux. On recueille d'autre part 45 kg de microfiltrat contenant 0,46 g/kg de lysozyme. Le rendement d'extraction en lysozyme est de 65,7%. Cette solution est ensuite ultrafiltrée de manière connue afin de concentrer le lysozyme. Le concentré obtenu est séché ou lyophilisé.

Exemple 2

5,28 kg de blanc d'œuf contenant 4,08 g/kg de lysozyme sont déchalazés et homogénéisés. Ils sont ensuite dilués dans 15,84 kg d'eau salée à 5 g/kg de chlorure de sodium. Le mélange est microfiltré sur un dispositif de microfiltration, comprenant un module de microfiltration SFEC M6-1000 d'une surface de membrane égale à 0,113 m$^2$ à une température de 42°C et une pression d'entrée de 2 bars, à une vitesse de circulation dans le module de microfiltration de 6 m/s. On réalise une diafiltration du mélange pendant 4 heures et on concentre le mélange jusqu'à l'obtention des 5,28 kg de blanc d'œuf initiaux. On recueille d'autre part 51,3 kg de microfiltrat contenant 0,32 g/kg de lysozyme. Le rendement d'extraction est de 76%. Le microfiltrat est ensuite concentré par ultrafiltration, de manière connue, puis séché ou lyophilisé.

Exemple 3

6,9 kg de blanc d'œuf déchalazés et homogénéisés sont additionnés de 13,3 kg d'eau salée à 5 g/kg et portés à 40°C. Le mélange est microfiltré sur un dispositif de microfiltration comprenant un module Norton équipé de 0,0248 m$^2$ de membranes céramiques ayant une porosité de 0,5 µ. La pression d'entrée est de 2,5 bars et la pression de sortie de 0,5 bar. La vitesse de recirculation est de 8 m/s. Le volume de liquide dans la cuve de recirculation est maintenu constant par recyclage du perméat. L'expérimentation dure 1 h 45. Le débit de microfiltration varie de 27 l.h$^{-1}$.m$^{-2}$ à 23 l.h$^{-1}$.m$^{-2}$. La teneur en lysozyme du liquide retenu par les membranes de microfiltration est de 2,14 g/kg. La teneur en lysozyme du microfiltrat est de 1 g/kg. La performance d'extraction du lysozyme dans les conditions utilisées varie donc de 23 à 27 g/h/m$^2$. La pureté en lysozyme dans le microfiltrat de fin d'expérimentation est voisine de 99%.

Exemple 4

4 kg d'entier d'œuf contenant 3,27 g/kg de lysozyme sont déchalazés et homogénéisés. Ils sont ensuite dilués dans 16 kg d'eau salée à 5 g/kg de chlorure de sodium. Le mélange est microfiltré sur un dispositif de microfiltration SFEC M6-4 d'une surface de membrane égale à 0,113 m$^2$, à une température de 44°C et une pression d'entrée de 2 bars, à une vitesse de circulation dans le module de microfiltration de 6 m/s. On réalise une diafiltration du mélange pendant 4 heures et on concentre le mélange jusqu'à l'obtention des 4 kg d'entier d'œuf initiaux. On recueille d'autre part 50,7 kg de microfiltrat contenant 0,13 g/kg de lysozyme. La pureté du lysozyme dans le microfiltrat est supérieure à 94%. Le rendement d'extraction en lysozyme est de 50%.

Exemple 5

880 kg de blanc d'œuf contenant 4,5 g/kg de lysozyme sont déchalazés et homogénéisés. Ils sont ensuite dilués dans 1750 kg d'eau salée à 5 g/kg de chlorure de sodium. Le mélange est microfiltré sur une installation industrielle de microfiltration comprenant un module SFEC de type M6-4 ayant une surface membranaire égale 5,7 m$^2$.

Les conditions opératoires sont les suivantes:
– température de microfiltration: 40°C

– pression d'entrée dans le module de microfiltration: 2 à 3 bars
– vitesse de circulation dans le module de microfiltration: 4 m/s.

On réalise, dès le début de la microfiltration, une diafiltration du blanc d'œuf dilué. L'expérimentation dure 5 heures. La teneur en lysozyme du microfiltrat moyen est de 0,8 g/kg. La performance d'extraction moyenne du lysozyme est de 24 g/h/m². La pureté du lysozyme dans le microfiltrat moyen est de 80%.

### Exemple 6

310 kg de blanc d'œuf contenant 4,21 g/kg de lysozyme sont préalablement déchalazés et homogénéisés. Ils sont ensuite dilués dans 750 kg d'eau salée à 5 g/kg de chlorure de sodium. Le mélange est microfiltré sur une installation industrielle de microfiltration comprenant un module SFEC de type M6-4 ayant une surface membranaire égale à 5,7 m².

Les conditions opératoires sont les suivantes:
– température de microfiltration: 42°C
– pression d'entrée dans le module de microfiltration: 2 à 3 bars
– vitesse de circulation dans le module de microfiltration: 5 m/s.

On concentre le blanc d'œuf dilué jusqu'à l'obtention de 124 kg de blanc d'œuf concentré d'une teneur en extrait sec de 26%. La teneur en lysozyme du microfiltrat est de 0,56 g/kg.

Le rendement en extraction du lysozyme dans le microfiltrat moyen est de 40%.

### Revendications

1. Procédé pour l'obtention de lysozyme à partir d'une matière première à base de blanc d'œuf, caractérisé en ce qu'on soumet une matière première fluide base de blanc d'œuf à au moins une microfiltration, par mise en contact de ladite matière sous pression et sous flux tangentiel avec une membrane capable de séparer les particules ayant une taille comprise entre 0,02 micron et 10 microns environ, ce qui fournit d'une part un microfiltrat passant à travers la membrane et contenant au moins une fraction du lysozyme de la matière première et d'autre part un produit retenu par la membrane, qui conserve les propriétés naturelles de la matière première à base de blanc d'œuf mais est appauvri en lysozyme.

2. Procédé selon la revendication 1, caractérisé en ce que la matière première à base de blanc d'œuf est le blanc d'œuf lui-même et/ou l'entier d'œuf.

3. Procédé selon l'une des revendications 1 ou 2, caractérisé en ce que la matière première à base de blanc d'œuf est diluée au préalable avec de l'eau contenant des sels ou sucres solubles, tels que, respectivement le chlorure de sodium et le glucose ou le saccharose.

4. Procédé selon l'une quelconque des revendications 1 à 3, caractérisé en ce que la matière première à base de blanc d'œuf est additionnée d'une substance antioxydante, telle que l'acide ascorbique.

5. Procédé selon l'une quelconque des revendications 1 à 4, caractérisé en ce que, lors de la mise en œuvre de la microfiltration, on réalise une diafiltration de la matière à base de blanc d'œuf en lui ajoutant en continu une quantité d'eau égale à la quantité de microfiltrat sortant.

6. Procédé selon l'une quelconque des revendications 1 à 5, caractérisé en ce que le microfiltrat contenant le lysozyme est concentré et purifié par ultrafiltration de manière connue puis déshydraté par lyophilisation ou séchage par atomisation.

### Claims

1. Process for obtaining lysozyme from a starting material based on white of egg, characterised in that a fluid starting material based on white of egg is subjected to at least one microfiltration by bringing the said material into contact, under pressure and in tangential flow, with a membrane capable of separating off the particles having a size of, approximately, between 0.02 micron and 10 microns, thereby giving, firstly, a microfiltrate which passes through the membrane and contains at least a fraction of the lysozyme of the starting material and, secondly, a product which is held back by the membrane and retains the natural properties of the starting material based on white of egg but is depleted in lysozyme.

2. Process according to Claim 1, characterised in that the starting material based on white of egg is white of egg itself and/or whole of egg.

3. Process according to either of Claims 1 and 2, characterised in that the starting material based on white of egg is diluted beforehand with water containing soluble salts or soluble sugars such as, respectively, sodium chloride and glucose or sucrose.

4. Process according to any one of claims 1 to 3, characterised in that an antioxidant, such as ascorbic acid is added to the starting material based on white of egg.

5. Process according to any one of claims 1 to 4, characterised in that during the performance of the microfiltration, a diafiltration of the material based on white of egg is carried out by continuously adding thereto an amount of water equal to the amount of microfiltrate which issues.

6. Process according to any one of Claims 1 to 5, characterised in that the microfiltrate containing the lysozyme is concentrated and purified by ultrafiltration in a known manner and then dehydrated by lyophilisation or spray-dried.

### Patentansprüche

1. Verfahren zur Gewinnung von Lysozym aus Eiweiss enthaltendem Ausgangsmaterial, dadurch gekennzeichnet, dass man ein Eiweiss enthaltendes flüssiges Ausgangsmaterial zumindest einer Mikrofiltration unterwirft, indem man das besagte Material unter Druck und unter einem tangentialen Fluss mit einer Membran in Kontakt bringt, die in der Lage ist, Partikel, die eine Grösse zwischen ungefähr 0,02 μm und 10 μm haben abzutrennen, so dass einerseits ein Mikrofiltrat erhalten wird, das die Membran passiert und wenigstens eine Fraktion des Lysozyms des Ausgangsmaterials enthält und andererseits ein durch die Membran zurückgehaltenes Produkt erhalten wird, das die natürlichen Eigenschaften des Eiweiss enthaltenden Ausgangsmaterials beibehält, aber an Lysozym verarmt ist.

2. Verfahren nach Anspruch 1, dadurch gekennzeichnet, dass das Eiweiss enthaltende Ausgangsmaterial das Eiweiss selbst und/oder das vollständige Ei ist.

3. Verfahren nach einem der Ansprüche 1 oder 2, dadurch gekennzeichnet, dass das Eiweiss enthaltende Ausgangsmaterial vorher mit Wasser verdünnt wird, das Salze oder lösliche Zucker enthält, wie bzw. Natriumchlorid, Glucose oder Saccharose.

4. Verfahren nach irgendeinem der Ansprüche 1 bis 3, dadurch gekennzeichnet, dass dem Eiweiss enthaltenden Ausgangsmaterial ein Antioxidans wie Ascorbinsäure zugefügt wird.

5. Verfahren nach irgendeinem der Ansprüche 1 bis 4, dadurch gekennzeichnet, dass man bei der Durchführung der Mikrofiltration eine Diafiltration des Eiweiss enthaltenden Materials realisiert, indem man diesem kontinuierlich Wasser in einer Menge hinzufügt, die gleich der Menge an Mikrofiltrat ist, die abläuft.

6. Verfahren nach irgendeinem der Ansprüche 1 bis 5, dadurch gekennzeichnet, dass das Mikrofiltrat, das Lysozym enthält, durch Ultrafiltration auf bekannte Weise konzentriert und gereinigt wird und dann durch Gefriertrocknung oder Sprühtrocknung entwässert wird.